# EUROPÄISCHE PATENTANMELDUNG

(11) **EP 0 988 847 A2**
(43) Veröffentlichungstag der Anmeldung: **29.03.2000**
(21) Anmeldenummer: 99118793.1
(22) Anmeldetag: 23.09.1999
(51) Int. Cl.: A61F 15/00

(54) **Tampon und Tampon-Packung**

(30) Priorität: 24.09.1998 DE 19843963
(71) Anmelder: RAUSCHER & CO., VERBANDSTOFF- UND WATTEFABRIKEN GESELLSCHAFT M.B.H., 2525 Schöngau/Tr. (AT)
(72) Erfinder: Leuprecht, Helmut, Dipl.-Ing., 1130 Wien (AT)
(74) Vertreter: Patentanwälte Leinweber & Zimmermann

(57) **Zusammenfassung**

Bei einem Tampon mit einem in eine Körperhöhle, insbesondere in die weibliche Scheide, einführbaren Saugelement aus einem zum Aufnehmen von Körperflüssigkeit geeigneten Material und einem an dem Saugelement festgelegten Rückholfaden, bei dem das Saugelement zumindest teilweise mit einem Wirkstoff, wie etwa einem Gleitmittel, versetzt ist, wird vorgeschlagen, den Rückholfaden zumindest teilweise mit einem den Wirkstoff abweisenden Mittel zu versetzen.

## Beschreibung

Die Erfindung betrifft einen Tampon mit einem in eine Körperhöhle, insbesondere in die weibliche Scheide, einführbaren Saugelement aus einem zum Aufnehmen von Körperflüssigkeit geeigneten Material und einem an dem Saugelement festgelegten Rückholfaden, wobei das Saugelement zumindest teilweise mit einem Wirkstoff, wie etwa einem Gleitmittel, versetzt ist, sowie eine entsprechende Tampon-Packung.

Derartige Tampons sind beispielsweise aus der US 3,428,044 und der EP 0 697 202 A2 bekannt und können durch Wahl eines geeigneten Wirkstoffs insbesondere bei trockenem Scheidengang zur Prophylaxe von Infektionen eingesetzt werden. In der EP 0 697 202 A2 wird dazu vorgeschlagen, das Saugelement mit einem hydrophoben Gleitmittel, wie etwa Vaseline-Öl zu versetzen, um so das Eindringen von möglicherweise mit Keimen kontaminiertem Wasser in den Scheidengang während des Badens oder Schwimmens zu verhindern.

Es hat sich jedoch gezeigt, daß selbst bei sachgemäßer Benutzung der bekannten Tampons geringe Mengen Flüssigkeit aus der Körperhöhle austreten. Das kann neben weiteren Unannehmlichkeiten insbesondere auch zur Verschmutzung der Bekleidung, insbesondere Badebekleidung, führen.

Angesichts dieser Probleme im Stand der Technik liegt der Erfindung die Aufgabe zugrunde, einen Tampon der vorstehend beschriebenen Art bereitzustellen, mit dem das Austreten von Flüssigkeit aus der Körperhöhle zuverlässig verhindert werden kann, und eine entsprechende Tampon-Packung anzugeben.

Hinsichtlich des Tampons wird diese Aufgabe durch eine Weiterbildung der bekannten Tampons gelöst, die im wesentlichen dadurch gekennzeichnet ist, daß der Rückholfaden zumindest teilweise mit einem den Wirkstoff abweisenden Mittel versetzt ist.

Überraschenderweise hat es sich gezeigt, daß die selbst bei sachgemäßer Benutzung der bekannten Tampons aus der Körperhöhle austretende Flüssigkeit überwiegend aus dem bei Erwärmung auf Körpertemperatur flüssig, zumindest dickflüssig werdenden Wirkstoff besteht, der im flüssigen Zustand über den Rückholfaden, insbesondere durch die Kapillarwirkung herkömmlicher Rückholfäden, aus der das Saugelement aufnehmenden Körperhöhle geleitet wird. Mit dem erfindungsgemäß weitergebildeten Tampon wird diese Ableitung des auf Körpertemperatur erwärmten Wirkstoffs zuverlässig verhindert, indem der zum Entfernen des Saugelementes benötigte und während der Benutzung des Tampons außerhalb der Körperhöhle freiliegende Rückholfaden mit einem den Wirkstoff abweisenden und dadurch dessen Ableitung aus der Körperhöhle verhindernden Mittel versetzt wird.

Wie eingangs bereits erläutert, hat es sich insbesondere bei Verwendung eines erfindungsgemäßen Tampons während des Badens oder Schwimmens als besonders zweckmäßig erwiesen, wenn der Wirkstoff hydrophob ist, beispielsweise Vaseline-Öl aufweist. Dazu kann die Oberfläche des Saugelementes zumindest teilweise mit dem Wirkstoff imprägniert werden. Diese Imprägnierung kann beispielsweise dadurch erfolgen, daß der Wirkstoff auf das Saugelement aufgetragen wird, wobei das Saugelement bereits in einer entsprechenden Packung eingelegt sein kann.

Insbesondere bei Einsatz eines Vaseline-Öl aufweisenden Wirkstoffs kann eine vorzugsweise wässrige Emulsion eines Harzes als den Wirkstoff abweisendes Mittel eingesetzt werden. Zur Herstellung eines erfindungsgemäßen Tampons kann der üblicherweise im wesentlichen aus einem Baumwollgarn bestehende Rückholfaden zumindest teilweise mit dem den Wirkstoff abweisenden Material imprägniert werden.

Zum Transport und zur Lagerung hat es sich als besonders zweckmäßig erwiesen, wenn der erfindungsgemäße Tampon in einer Packung mit mindestens einer das Saugelement aufnehmenden Kammer und einem lösbar daran festgelegten und diese Kammer verschließenden Schließelement aufgenommen ist. Derartige üblicherweise in Form von Blisterpackungen gebildete Packungen sind beispielsweise aus der WO 81/02251 bekannt. Bei der in dieser Schrift angegebenen Packung ist der Wirkstoff in einer separaten Kammer der Packung angeordnet und wird erst nach Öffnen der Packung auf das Saugelement aufgebracht. Eine derartige Vorbereitung des Saugelements vor dem bestimmungsgemäßen Gebrauch ist nicht nur umständlich, sondern kann auch zu einer die gewünschte Wirkung beeinträchtigenden ungleichmäßigen Verteilung des Wirkstoffs auf der Oberfläche des Saugelementes führen. Daher ist es erfindungsgemäß besonders bevorzugt, wenn das in der Kammer der Packung aufgenommene Saugelement bereits mit dem Wirkstoff versetzt, vorzugsweise imprägniert ist. Dabei kommt es jedoch zu dem Problem, daß der an dem Saugelement festgelegte Rückholfaden während der Lagerung und dem Transport erfindungsgemäßer Tampons mit dem Wirkstoff kontaminiert werden kann, was insbesondere bei einem Wirkstoff in Form eines Gleitmittels zu Unannehmlichkeiten bei der Entnahme des Tampons aus der Packung führen kann.

Zur Lösung dieses Problems im Stand der Technik ist die erfindungsgemäße Tampon-Packung mit einer weiteren, den Rückholfaden aufnehmenden Kammer versehen. Auf diese Weise kann ein direkter Kontakt zwischen dem mit dem Wirkstoff versetzten Saugelement und dem in einer separaten Kammer aufgenommenen Rückholfaden verhindert werden. Dabei kann die Kontaminierung des einerseits an dem Saugelement festgelegten und andererseits in der weiteren Kammer aufgenommenen Rückholfadens besonders zuverlässig verhindert werden, wenn die weitere Kammer über eine Rille mit der das Saugelement aufnehmenden Kammer verbunden ist und der im übrigen in der weiteren Kammer aufgenommene Rückholfaden lösbar in dieser Rille festgelegt, vorzugsweise eingeklemmt ist.

Zur Vermeidung einer Verschmutzung des Rückholfadens während des Transports oder der Lagerung erfindungsgemäßer Packungen hat es sich als besonders zweckmäßig erwiesen, wenn auch die weitere Kammer mit dem Schließelement verschlossen ist.

Produktionstechnisch hat es sich als besonders vorteilhaft erwiesen, wenn die erfindungsgemäße Packung in Form einer sog. Blisterpackung verwirklicht wird, bei der die das Saugelement aufnehmende Kammer, die weitere, den Rückholfaden aufnehmende Kammer und ggf. die Rille in Form einer tiefgezogenen Folie, vorzugsweise einer tiefgezogenen Kunststoffolie, gebildet sind und das Schließelement eine an einem die Kammern umlaufenden Rand der tiefgezogenen Folie befestigte Deckfolie aufweist. Dabei kann die Deckfolie beispielsweise in Form einer an den Rändern der tiefgezogenen Folie dicht verschweißten Alufolie gebildet sein.

Ergonomische Untersuchungen haben gezeigt, daß die vorstehend beschriebenen Blisterpackungen zur Entnahme des Tampons besonders bevorzugt im Bereich der das Saugelement enthaltenden Kammer gehalten werden. Bei dieser Haltung kann die Lösung der Deckfolie von der tiefgezogenen Folie besonders einfach bewirkt werden, wenn die Deckfolie im Bereich der das Saugelement enthaltenden Kammer einen Öffnungsabschnitt aufweist. Dieser Öffnungsabschnitt kann beispielsweise in Form einer leicht von dem Rand der tiefgezogenen Folie abschälbaren oder zusammen mit einer Ecke der tiefgezogenen Folie von dem Rest der tiefgezogenen Folie lösbaren Aufreißecke gebildet sein.

Andererseits ist es besonders zweckmäßig, wenn zur Entnahme des Tampons zunächst die den Rückholfaden enthaltende Kammer geöffnet wird, um so die Entnahme des Tampons ohne Kontaminierung mit dem Wirkstoff durch Ergreifen des Rückholfadens zu ermöglichen. Unter diesem Gesichtspunkt ist es besonders zweckmäßig, wenn die Deckfolie im Bereich der den Rückholfaden enthaltenden weiteren Kammer einen beispielsweise ebenfalls in Form einer Aufreißecke gebildeten Öffnungsabschnitt aufweist.

Insbesondere bei Einsatz von Packungen mit einer Mehrzahl von durch entsprechende Trennlinien, wie etwa Perforationen, miteinander verbundenen Packungsbereichen, von denen jeder eine das Saugelement aufnehmende Kammer, eine den Rückholfaden aufnehmenden Kammer und ggf. eine diese Kammern miteinander verbindende Rille aufweist, hat es sich aus Platzgründen als besonders zweckmäßig erwiesen, wenn die das Saugelement aufnehmende Kammer und die den Rückholfaden aufnehmende weitere Kammer längs einer gemeinsamen Geraden angeordnet sind und die Trennlinien parallel zu den entsprechenden Geraden verlaufen. Im Hinblick auf die Tatsache, daß derartige Packungen zum Entnehmen des Tampons vorzugsweise mit der linken Hand gehalten werden, während die Decktolle mit der rechten Hand gelöst wird, hat es sich aus ergonomischen Gründen als besonders zweckmäßig erwiesen, wenn mindestens ein Öffnungsabschnitt bei Ansicht der Packung längs der gemeinsamen Geraden in Richtung auf die das Saugelement aufnehmende Kammer auf der rechten Seite dieser Kammer angeordnet ist. Andererseits kann eine Kontaminierung des Benutzers mit dem Wirkstoff unter gleichzeitiger Sicherstellung einer ergonomisch günstigen Handhabung mit dieser Packungsanordnung sichergestellt werden, wenn mindestens ein Öffnungsabschnitt bei Ansicht der Packung längs der gemeinsamen Geraden in Richtung auf die den Rückholfaden enthaltende Kammer auf der rechten Seite dieser Kammer angeordnet ist.

Falls die Abdeckolie der Packung auf die tiefgezogene Folie aufgesiegelt ist, kann anstelle der Aufreißecke auch ein Öffnungsabschnitt in Form einer siegelfreien Zone vorgesehen sein. Diese siegelfreie Zone kann eine Breite von 1 bis 2 mm aufweisen und an einem Ende der Blisterverpackung angebracht sein oder die Blisterverpackung vollständig umlaufen. Dann kann die Abdeckfolie an allen Seiten ergriffen und von der tiefgezogenen Folie abgezogen werden.

Wie der vorstehenden Erläuterung des erfindungsgemäßen Tampons bereits zu entnehmen ist, zeichnet sich ein Verfahren zum Herstellen einer diesen Tampon enthaltenden Packung, bei dem der Tampon in eine entsprechende Kammer der Packung eingelegt, mit einem Wirkstoff versetzt und die Kammer mit einem Schließelement verschlossen wird, im wesentlichen dadurch aus, daß der Tampon erst nach Einlegen in die Kammer mit dem Wirkstoff versetzt wird. Dadurch wird erreicht, daß auch beim Vorgang des Einlegens des Tampons in die Packung eine Kontamination des Rückholfadens mit dem Wirkstoff ausgeschlossen wird, weil der Wirkstoff zu diesem Zeitpunkt noch gar nicht auf den Tampon aufgebracht ist. In diesem Zusammenhang ist es besonders zweckmäßig, wenn der Rückholfaden bereits vor Einlegen des Tampons in die Kammer von dem Tampon getrennt und in die weitere Kammer der Packung eingelegt wird.

Das Aufbringen des Wirkstoffs auf dem in die Kammer eingelegten Tampon kann besonders einfach und gleichmäßig erfolgen, wenn der Wirkstoff auf den in die Kammer eingelegten Tampon aufgesprüht wird.

In fertigungstechnischer Hinsicht hat es sich als zweckmäßig erwiesen, wenn die Packung mit dem darin eingelegten Tampon in Richtung auf eine stationäre Einrichtung zum Aufbringen des Wirkstoffs, wie etwa eine Aufsprühstation transportiert wird. Dabei kann ein besonders zuverlässiger Betrieb verwirklicht werden, wenn der Transport der Packung derart gesteuert wird, daß die Packung vor Erreichen der Aufsprühstation gestoppt wird, bis die zuvor in der Aufsprühstation behandelte Packung die Aufsprühstation verlassen hat, und die Packung erst danach in die Aufsprühstation eingefahren wird. Dabei kann der Wirkstoff bereits während des Transports in die Aufsprühstation auf den Tampon aufgebracht werden, um den Tampon so über die gesamte Länge mit dem Wirkstoff zu besprühen.

In den meisten Fällen ist es besonders bevorzugt, wenn der Großteil des Wirkstoffs, wie etwa des Gleitmittels, auf den Kuppenteil des Tampons aufgebracht wird. Danach kann die Packung innerhalb der Aufsprühstation gestoppt und die restliche Wirkstoffmenge entsprechend auf den Kuppenteil aufgebracht werden. Nach Beendigung des Aufsprühvorganges wird die Packung aus der Aufsprühsstation ausgefahren und die nächste Packung eingefahren.

In einer besonders vorteilhaften Ausführungsform der Erfindung kann die Aufsprühstation einen Drucktank mit Auslaßventilen aufweisen, wobei die Menge des auf die einzelnen Tampons aufgesprühten Wirkstoffes über den Druck in dem Drucktank, die Aufsprühzeit und den Kolbenhub des Auslaßventiles gesteuert wird. Nach Verlassen der Aufsprühstation kann die Packung an eine an sich bekannte Siegelmaschine übergeben werden, in der die den Tampon und den Rückholfaden enthaltenden Kammern der Packung mit einer Deckfolie, wie etwa einer Alufolie verschlossen werden.

Bei einer eine Mehrzahl von durch Trennlinien miteinander verbundenen Packungsbereichen aufweisenden Packung kann zum Schluß des Herstellungsvorganges eine Übergabe an eine Rollenstanze erfolgen, in der die Trennlinien in Form von entprechenden Perforationen ausgebildet werden.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- Fig. 1: eine Draufsicht auf eine erfindungsgemäße Packung mit einen darin aufgenommenen erfindungsgemäßen Tampon
- Fig. 2: eine Schnittansicht durch die Packung nach Fig. 1 längs der in Fig. 1 angegebenen Schnittebene a-a und
- Fig. 3: eine Draufsicht auf eine zweite Ausführungsform einer erfindungsgemäßen Packung.

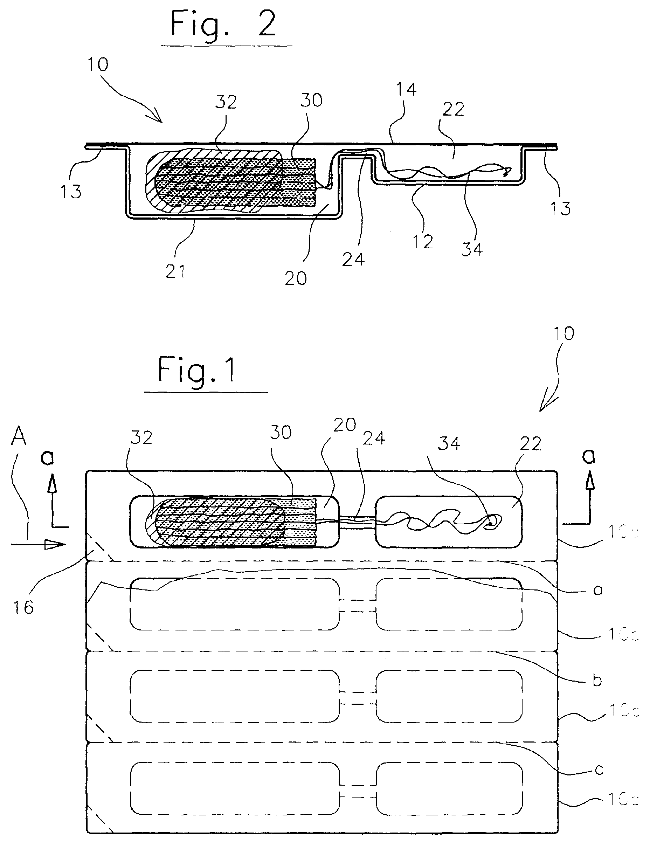

Die in den Fig. 1 und 2 dargestellte Packung besteht im wesentlichen aus einer tiefgezogenen Kunststoffolie 12 und einer auf dem oberen Rand 13 dieser Kunststoffolie dicht aufgeschweißten dünnen Alu-Deckfolie 14. Die Packung weist vier über parlallele Trennlinien in Form von geradlinig verlaufenden Perforationen a, b, c miteinander verbundene Packungsbereiche 10a, 10b, 10c und 10d auf. Jeder Packungsbereich umfaßt eine muldenförmige Kammer 20 und eine über eine Rille 24 damit verbundene weitere, kleinere muldenförmige Kammer 22.

In der größeren Kammer 20 ist ein Saugelement 30 eines Tampons aufgenommen, das zumindest im Bereich der Einführspitze mit einen Gleitmittel in Form von Vaseline-Öl versetzt ist. An dem Saugelement 30 ist ein über die Rille 24 in die kleinere Kammer 22 geführter Rückholfaden 34 festgelegt. Die Seitenwände der Kammer 24 weisen einen sich in Richtung auf den Boden 21 der Kammer 20 verringernden Abstand auf, so daß der Rückholfaden 34 lösbar zwischen den Seitenwänden der Rille 24 eingeklemmt werden kann. Zur Vermeidung der Ableitung des bei Temperaturerhöhung dünnflüssigen Vaseline-Öls aus der das Saugelement 30 aufnehmenden Körperhöhle ist der Rückholfaden 34 mit einer wässrigen Emulsion eines das Vaseline-Öl abweisenden Harzes imprägniert.

Wie besonders deutlich anhand der Fig. 2 zu erkennen ist, sind die das Saugelement 30 und den Rückholfaden 34 aufnehmenden Kammern 20 und 22 und ebenso wie die Rille 24 mit der Deckfolie 14 verschlossen. Weiter ist den Fig. 1 und 2 zu entnehmen, daß die Kammern 20 und 22 jedes Packungsbereichs 10a, 10b, 10c und 10d längs einer gemeinsamen Geraden angeordnet und etwa quaderförmig mit etwa gleicher Breite gebildet sind, wobei die Rille 24 längs der Seitenhalbierenden der einander zugewandten Seiten der Kammern 20 und 22 verläuft.

Zum Entnehmen eines Tampons aus der in den Fig. 1 und 2 dargestellten Packung wird zunächst der entsprechende Packungsbereich von den anderen Packungsbereichen längs der entsprechenden Perforation getrennt. Anschließend wird die Deckfolie 14 von der tiefgezogenen Kunststoffolie 12 gelöst und der Tampon an dem Rückholfaden 34 ergriffen und aus der Packung entnommen. Wie eingangs bereits erläutert, hat es sich als ergonomisch besonders günstig erwiesen, wenn die Packung zum Lösen der Deckfolie 14 von der tiefgezogenen Folie 12 mit der linken Hand gehalten wird, während die rechte Hand die Deckfolie ergreift und löst. Zu diesem Zweck ist jeder Packungsbereich 10a, 10b, 10c und 10d mit einer bei Ansicht der Packung längs der gemeinsamen Geraden in Richtung auf die das Saugelement aufnehmende Kammer 20, wie durch den Pfeil A dargestellt, an der vorderen rechten Ecke des Packungsbereichs angeordneten Aufreißecke versehen, in der die Deckfolie leicht von dem Rand 13 der tiefgezogenen Folie 12 abgeschält und zum weiteren Lösen von der tiefgezogenen Folie 12 ergriffen werden kann.

Die Herstellung der in der Zeichnung dargestellten Packung kann auf einer Tiefziehpackungsmaschine erfolgen, wobei die Befüllung sowohl manuell als auch maschinell direkt von der Tamponmaschine erfolgen kann.

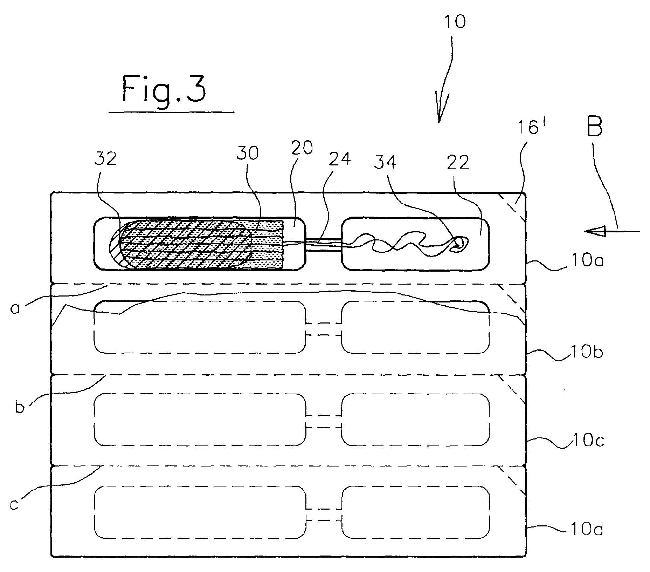

Die in Fig. 3 dargestellte Ausführungsform der Erfindung entspricht im wesentlichen der anhand der Fig. 1 und 2 dargestellten Ausführungsform. Bei dieser Ausführungsform sind die Aufreißecken 16' der Deckfolie 14 jedoch im Bereich der den Rückholfaden 34 aufnehmenden Kammer 22 angeordnet, und zwar bei Ansicht der Packung längs der gemeinsamen Geraden in Richtung auf die den Rückholfaden 34 enthaltende Kammer 22, wie durch den Pfeil B angedeutet, an der vorderen rechten Ecke der einzelnen Packungsbereiche 10a, 10b, 10c und 10d. Mit dieser Anordnung der Aufreißecken 16' wird erreicht, daß bei Lösen der Deckfolie von der tiefgezogenen Folie zunächst die den Rückholfaden 34 enthaltende Kammer 22 geöffnet wird. In diesem Zustand kann der Rückholfaden 34 ohne Kontaminierung durch das auf der Spitze des Saugelementes 30 aufgebrachte Vaseline-Öl 32 ergriffen werden.

Die Erfindung ist nicht auf die anhand der Zeichnung erläuterten Ausführungsbeispiele beschränkt. Vielmehr ist auch daran gedacht, jeden Packungsbereich sowohl mit einer Aufreißecke im Bereich der das Saugelement enthaltenen Kammer 20, als auch mit einer Aufreißecke im Bereich der den Rückholfaden 34 enthaltenden Kammer 22 zu versehen. Ferner kann die erfindungsgemäße Packung auch mehr oder weniger als vier Packungsbereiche aufweisen. Darüber hinaus können die erfindungsgemäßen Tampons nicht nur, wie in der Zeichnung dargestellt, über etwa 2/3 des Saugelementes mit dem Wirkstoff versetzt sein, sondern über die gesamte Fläche des Saugelementes.

## Patentansprüche

1. Tampon mit einem in eine Körperhöhle, insbesondere in die weibliche Scheide, einführbaren Saugelement (30) aus einem zum Aufnehmen von Körperflüssigkeit geeigneten Material und einem an dem Saugelement (30) festgelegten, vorzugsweise im wesentlichen aus einem Baumwollgarn bestehenden Rückholfaden (34), wobei das Saugelement (30) zumindest teilweise mit einem vorzugsweise hydrophoben Wirkstoff, wie etwa einem Gleitmittel (32), beispielsweise Vaseline-Öl versetzt ist, wobei die Oberfläche des Saugelements (30) vorzugsweise zumindest teilweise mit dem Wirkstoff imprägniert ist, dadurch gekennzeichnet, daß der Rückholfaden (34) zumindest teilweise mit einem den Wirkstoff abweisenden Mittel wie etwa einer vorzugsweise wässrigen Emulsion eines Harzes versetzt, vorzugsweise zumindest teilweise mit dem den Wirkstoff abweisenden Material imprägniert ist.

2. Packung für Tampons nach Anspruch 1 mit mindestens einer das Saugelement (30) aufnehmenden Kammer (20) und einem lösbar daran festgelegten und die Kammer verschließenden Schließelement (14), dadurch gekennzeichnet, daß der Rückholfaden (34) in einer weiteren Kammer (22) der Packung (10) aufgenommen ist.

3. Packung nach Anspruch 2, dadurch gekennzeichnet, daß die weitere Kammer (22) über eine Rille (24) mit der das Saugelement (30) aufnehmenden Kammer (20) verbunden ist.

4. Packung nach Anspruch 3, dadurch gekennzeichnet, daß der Rückholfaden (34) in der Rille lösbar festgelegt, vorzugsweise eingeklemmt ist.

5. Packung nach einem der Ansprüche 2 bis 4, dadurch gekennzeichnet, daß die weitere Kammer (22) mit dem Schließelement (14) verschlossen ist.

6. Packung nach einem der Ansprüche 2 bis 5, dadurch gekennzeichnet, daß die das Saugelement (30) aufnehmende Kammer (20), die weitere Kammer (22) und ggf. die Rille (24) in Form einer tiefgezogenen Folie gebildet sind und das Schließelement (14) eine an einem die Kammern (20, 22) umlaufenden Rand (13) der tiefgezogenen Folie (12) befestigte insbesondere aufgesiegelte Deckfolie (14) aufweist.

7. Packung nach Anspruch 6, dadurch gekennzeichnet, daß die Deckfolie (14) eine im Bereich der das Saugelement (30) enthaltenden Kammer (20) einen beispielsweise in Form einer Aufreißecke (16) gebildeten Öffnungsabschnitt aufweist.

8. Packung nach Anspruch 6 oder 7, dadurch gekennzeichnet, daß die Deckfolie (14) im Bereich der den Rückholfaden (34) enthaltenden weiteren Kammer (22) einen beispielsweise in Form einer Aufreißecke (16') gebildeten Öffnungsabschnitt aufweist.

9. Packung nach Anspruch 7 oder 8, dadurch gekennzeichnet, daß die das Saugelement (30) aufnehmende Kammer und die den Rückholfaden (34) aufnehmende weitere Kammer (22) längs einer gemeinsamen Geraden angeordnet sind.

10. Packung nach Anspruch 9, dadurch gekennzeichnet, daß mindestens ein Öffnungsabschnitt (16) bei Ansicht der Packung längs der gemeinsamen Geraden in Richtung auf die das Saugelement (30) enthaltende Kammer (20) auf der rechten Seite dieser Kammer (20) angeordnet ist.

11. Packung nach Anspruch 9 oder 10, dadurch gekennzeichnet, daß mindestens ein Öffnungsabschnitt (16') bei Ansicht der Packung längs der gemeinsamen Geraden in Richtung auf die den Rückholfaden (34) enthaltende Kammer (22) auf der rechten Seite dieser Kammer (22) angeordnet ist.

12. Packung nach einem der Ansprüche 2 bis 11, gekennzeichnet durch eine Mehrzahl von durch Trennlinien (a, b, c) miteinander verbundenen Packungsbereichen (10a, 10b, 10c, 10d), von denen jede eine das Saugelement (30) aufnehmende Kammer (20), eine den Rückholfaden (34) aufnehmende Kammer (22) und ggf. eine diese Kammer (20, 22) miteinander verbindende Rille (24) aufweist.

13. Verfahren zum Herstellen einer einen Tampon nach Anspruch 1 enthaltenden Packung, insbesondere nach einem der Ansprüche 2 bis 12, bei dem der Tampon in eine entsprechende Kammer der Packung eingelegt, mit einem Wirkstoff versetzt und die Kammer mit einem Schließelement verschossen wird, dadurch gekennzeichnet, daß der Tampon erst nach Einlegen in die Kammer mit dem Wirkstoff versetzt wird.

14. Verfahren nach Anspruch 13, dadurch gekennzeichnet, daß der Rückholfaden vor Einlegen des Tampons in die Kammer von dem Tampon getrennt und in die weitere Kammer eingelegt wird.

15. Verfahren nach Anspruch 13 oder 14, dadurch gekennzeichnet, daß der Wirkstoff auf den in die Kammer eingelegten Tampon aufgesprüht wird.

16. Verfahren nach den Ansprüchen 13 bis 15, dadurch gekennzeichnet, daß die Packung mit einem darin eingelegten Tampon in Richtung auf eine stationäre Einrichtung zum Aufbringen des Wirkstoffs transportiert wird.

17. Verfahren nach Anspruch 16, dadurch gekennzeichnet, daß der Transport der Packung vor Erreichen der Aufbringeinrichtung gestoppt wird, bis die vorherige Packung die Aufbringeinrichtung verlassen hat und die Packung erst danach in die Aufbringeinrichtung transportiert wird.

18. Verfahren nach Anspruch 16 oder 17, dadurch gekennzeichnet, daß der Wirkstoff bereits während des Transports der Packung in die Aufbringeinrichtng auf den Tampon aufgebracht wird.
